# EUROPÄISCHE PATENTANMELDUNG

(11) **EP 1 300 393 A1**
(43) Veröffentlichungstag der Anmeldung: **09.04.2003**
(21) Anmeldenummer: 01123293.1
(22) Anmeldetag: 05.10.2001
(51) Int. Cl.: C07C 265/00, C07C 265/04

(54) **Isocyanato-Isocyano-Verbindungen**

(71) Anmelder: Dr. Eckert GmbH, 85399 Hallbergmoos (DE)
(72) Erfinder: Eckert, Heiner, Dr., 85399 Hallbergmoos (DE); Antony, Andreas, Dr., 85737 Ismaning (DE)
(74) Vertreter: HOFFMANN - EITLE

(57) **Zusammenfassung**

Die vorliegende Erfindung betrifft Isocyanato-isocyano-Verbindungen als multifunktionelle Verbindungen mit hochreaktiven Funktionen unterschiedlicher Selektivität, Verfahren zur Herstellung dieser Verbindungen sowie die Verwendung der Verbindungen in der chemischen Synthese.

## Beschreibung

Diese Erfindung betrifft Isocyanato-isocyano-Verbindungen als multifunktionelle Verbindungen mit hochreaktiven Funktionen unterschiedlicher Selektivität, Verfahren zur Herstellung dieser Verbindungen sowie die Verwendung der Verbindungen in der chemischen Synthese.

Der wirtschaftliche Erfolg einer chemischen Synthese hängt bei gegebenem Zielprodukt in erster Linie von der Anzahl der Stufen der Reaktionsfolge und den Ausbeuten der einzelnen Reaktionsstufen ab. So beträgt beispielsweise, eine mittlere Stufenausbeute von 70 % angenommen, bei einer 10-stufigen Synthese die Gesamtausbeute 2,8 %, bei einer 5-stufigen hingegen 16,8 %, also 6-mal soviel. Da Ausbeuten von 70 % kaum mehr wesentlich verbessert werde können, muß das Ziel einer Syntheseplanung sein, die Anzahl der Synthesestufen möglichst stark zu reduzieren. Erreicht werden kann dieses Ziel dadurch, daß man mehrere verschiedene funktionelle Gruppen nicht nacheinander, sondern gleichzeitig selektiv umsetzt. Dafür gibt es wiederum zwei Lösungen: Zum einen kann man mehrere Reagenzien in einer Multikomponentenreaktion (MCR) mit dem Ausgangsstoff umsetzen, zum anderen können bei geeigneter Wahl der funktionellen Gruppen des Ausgangsstoffs und/oder der Reagenzien mehrere unterschiedliche Reaktionen (gleichzeitig) an den verschiedenen Funktionen des Ausgangsstoffs selektiv ablaufen, d.h. Ausgangsstoff und/oder Reagenzien sind multifunktionell. Damit können Schutzgruppentechniken sowie Transformationen funktioneller Gruppen entfallen, womit die Stufenzahl stark reduziert und die Ausbeute erhöht werden kann.

Multifunktionelle Verbindungen sind beispielsweise Aminosäuren. An diesem allgemein bekannten System soll die Problemstellung beispielhaft erörtert werden. In einer Aminosäure ist die Carbonsäurefunktion nicht reaktiv genug, als daß weitere Reaktionen ohne weiteres ablaufen könnten. Erst durch eine Aktivierung kann sie zur Reaktion gebracht werden (H.-D. Jakubke, H. Jeschkeit, "Aminosäuren, Peptide, Proteine", Verlag Chemie, Weinheim, 1982, 110). Das heißt, zur Erlangung hinreichender Reaktivität ist eine zusätzliche Reaktionsstufe nötig. Um die Reaktion selektiv zu machen, muß außerdem die Aminofunktion geschützt sowie nach erfolgter Reaktion wieder entschützt werden, was zwei weitere Reaktionsstufen erfordert. Erst im Anschluß daran kann eine. selektive Reaktion an der Aminofunktion in Erwägung gezogen werden. In der Summe sind also drei zusätzliche Reaktionsstufen für die selektive Umsetzung beider Funktionen einer Aminosäure notwendig. Das gezeigte Reaktionsprinzip gilt natürlich nicht nur für Aminosäuren, sondern für alle Verbindungen mit wenigstens zwei funktionellen Gruppen mit orthogonaler (entgegengesetzter) Selektivität (z.B. elektrophiler und nucleophiler Natur).

Es ist daher eine Aufgabe der vorliegenden Erfindung, Verbindungen mit wenigstens zwei Typen von reaktiven funktionellen Gruppen bereitzustellen, die eine unterschiedliche Selektivität gegenüber Reagenzien besitzen.

Diese Aufgabe wurde überraschend durch die Verbindungen der folgenden allgemeinen Formel (I) gelöst:

(CN)ₐ-X-(NCO)_{b} (I)

worin a und b unabhängig voneinander eine ganze Zahl größer gleich 1 sind und X ausgewählt ist aus:
- geradkettigem oder verzweigtkettigem Alkyl, gegebenenfalls mit einer oder mehreren Gruppen in der Alkylkette, unabhängig ausgewählt aus Cycloalkyl, Aryl und Heterocyclyl,
- Cycloalkyl,
- Aryl und
- Heterocyclyl,
wobei X gegebenenfalls mit einer oder mehreren funktionellen Gruppen substituiert ist, die von Isocyano und Isocyanato verschieden sind.

In den Verbindungen der Formel (I) sind die Indizes a und b unabhängig voneinander bevorzugt eine ganze Zahl im Bereich von 1 bis 4, besonders bevorzugt von 1 bis 3, am meisten bevorzugt beide 1. Isocyanatogruppen und Isocyanogruppen können sich in den erfindungsgemäßen Verbindungen der Formel (I) allgemein in einer 1,1-Position oder höher zueinander befinden (z.B. in einer 1,1-, 1,2-, 1,3-, 1,4-, 1,5- etc. Position). Bevorzugt befinden sich Isocyanatogruppen und Isocyanogruppen in den Verbindungen der Formel (I) aus Stabilitätsgründen in einer 1,3-Position oder höher zueinander, besonders bevorzugt in einer 1,5-Position oder höher (z.B. in einer 1,5-, 1,6-, 1,7- etc. Position).

Alkyl und Cycloalkyl wie in dieser Beschreibung verwendet können sowohl gesättigtes als auch ungesättigtes Alkyl bzw. Cycloalkyl bedeuten. Wenn die zentrale Gruppe X der Verbindung der Formel (I) geradkettiges oder verzweigtkettiges Alkyl ist, kann dies in der Alkylkette eine oder mehrere Gruppen aufweisen, ausgewählt aus Cycloalkyl, Aryl und Heterocyclyl. So kann die Gruppe X z.B. eine Alkylcycloalkylgruppe oder eine Bis(alkyl)cycloalkylgruppe darstellen. In einer bevorzugten Ausführungsform der Erfindung ist X geradkettiges oder verzweigtkettiges C₃₋₂₅-Alkyl, besonders bevorzugt C₅₋₂₅-Alkyl, am meisten bevorzugt C₅-Alkyl. Exemplarisch können Methyl (mit Cyclalkyl, Aryl oder Heterocyclyl als Spacer-Gruppe), Ethyl, Propyl, Butyl, Pentyl und Hexyl genannt werden.

Wenn X in der Verbindung der allgemeinen Formel (I) Cycloalkyl ist oder (als Teil einer geradkettigen oder verzweigtkettigen Alkylgruppe) Cycloalkyl enthält, so ist dies in einer bevorzugten Ausführungsform der Erfindung C₃₋₁₀-Cycloalkyl, besonders bevorzugt C₅₋₈-Cycloalkyl, am meisten bevorzugt C₆-Cycloalkyl.

Wenn X oder ein Teil von X (in einer geradkettigen oder verzweigtkettigen Alkylgruppe) in der Verbindung der Formel (I) eine Arylgruppe ist, so kann dies eine mono, bi- oder polycyclische Arylgruppe sein, bevorzugt eine 6-gliedrige Arylgruppe, z.B. ein Phenylring.

Wenn X oder ein Teil von X (in einer geradkettigen oder verzweigtkettigen Alkylgruppe) in der Verbindung der Formel (I) eine Heterocyclylgruppe ist, so kann dies eine mono-, bioder polycyclische Gruppe sein, worin jeder Ring bevorzugt 4-, 5- oder 6-gliedrig ist und ein oder mehrere Heteroatome aus N, O und S, bevorzugt 1 bis 4 Heteroatome, enthält. Die Heterocyclylgruppe kann aromatisch (d.h. Heteroaryl) oder nicht-aromatisch sein und ist bevorzugt ausgewählt aus Indolyl, Imidazolyl, Oxazolyl, Oxazolinyl, Oxazolidinyl, Pyrrolyl, Chinolinyl, Pyrimidinyl, Piperazinyl, Piperidinyl, Furyl, Thiazolyl, Pyridinyl, Pyridazinyl, Pyrazinyl, Pyrrolidinyl, Pyrrazolyl, Triazinyl, Thiazolinyl, Thiazolidinyl, Azitidinyl, Guanyl, Adenyl, Cytidyl, Thyminyl, Uracilyl und Tetrazolyl.

Die erfindungsgemäße Verbindung der Formel (I) kann in X mit einer oder mehreren, von Isocyano und Isocyanato verschiedenen funktionellen Gruppen substituiert sein. Bevorzugt sind diese Gruppen unabhängig ausgewählt aus einer Estergruppe, einer Säureamidgruppe, einer Ketogruppe, einer Nitrogruppe, einem Halogen und einer Cyanogruppe. In einer Ausführungsform der Erfindung sind diese funktionellen Gruppen gegenüber Isocyano oder Isocyanato oder beiden nicht reaktiv. In einer anderen Ausführungsform kann allerdings eine solche Reaktivität selektiv gegenüber Isocyano oder Isocyanato oder gegenüber beiden bevorzugt sein, insbesondere dann, wenn die Verbindung der Formel (I) nach ihrer Herstellung direkt oder nach Aktivierung (z.B. durch Erwärmen) inter- oder intramolekular abreagieren soll.

Die erfindungsgemäßen Verbindungen der Formel (I) können durch verschiedene Verfahren hergestellt werden, die ebenfalls einen Teil der vorliegenden Erfindung bilden.

In einem ersten Verfahren (nachfolgend auch: Methode A) wird die Verbindung der Formel (I) hergestellt durch Phosgenierung einer Verbindung der folgenden allgemeinen Formel (II) :

(OHCHN)ₐ-X-(NH₂)_{b} (II)

worin a, b und X wie hier oben definiert sind. Die Verbindung der Formel (II) kann auch als Amino-formamido-Verbindung bezeichnet werden.

Die Phosgenierung wird in herkömmlicher, dem Fachmann bekannter Weise durchgeführt, d.h. mit Phosgen oder seinen höheren Homologen Diphosgen oder Triphosgen (s. z.B. I. Ugi, "Isonitrile Chemistry", Academic Press, New York, London, 1971). Die Reaktion wird üblicherweise in einem inerten Lösungsmittel und unter Verwendung einer Hilfsbase durchgeführt. Beispiele für einsetzbare Lösungsmittel sind Dichlormethan, Tetrahydrofuran, Dimethoxyethan, 1,4-Dioxan und Toluol. Die Hilfsbase kann, wie dem Fachmann allgemein bekannt, ein tertiäres Amin sein, z.B. Triethylamin. Besonders bevorzugt wird ein Überschuß an Hilfsbase verwendet, relativ zur Molmenge an Phosgen, um die Ausbeute der Reaktion zu steigern. Es wird vermutet, daß sich durch den Überschuß an Hilfsbase keine lokalen Säurekonzentrationen bilden können, so daß unerwünschte (Abbau-) Reaktionen zwischen Produkt der Formel (I) und Säure verhindert oder zumindest minimiert werden können. Als besonders vorteilhaft hat sich die Verwendung eines mindestens dreifachen Überschuß an Hilfsbase erwiesen, relativ zur Molmenge an Phosgen. Die Phosgenierungsreaktion wird vorzugsweise bei einer Temperatur im Bereich von -40 bis +40°C durchgeführt. Für den Fall, daß aromatische Isocyanatogruppen gebildet werden (X = Aryl oder Heteroaryl), beträgt die Temperatur besonders bevorzugt -20 bis +40°C, am meisten bevorzugt 0 bis 30°C, ansonsten besonders bevorzugt -40 bis +20°C, am meisten bevorzugt -20 bis 0°C.

In einem zweiten Verfahren (nachfolgend auch: Methode B) wird die Verbindung der Formel (I) hergestellt durch eine zweistufige Reaktion. In einem ersten Schritt erfolgt eine Thermolyse einer Verbindung der folgenden allgemeinen Formel (III) :

(OHCHN)ₐ-X-(CON₃)_{b} (III)

worin a, b und X wie oben für Formel (I) definiert sind. Die Verbindung der Formel (III) kann auch als Formamidocarbonsäureazid bezeichnet werden und ist handelsüblich oder kann durch dem Fachmann bekannte Standardreaktionen synthetisiert werden (s. z.B. Organikum, 20. Aufl., Johann Ambrosius Barth Verlag, Heidelberg, Berlin, 1996, S. 620-621). Die Thermolyse führt über einen Curtius-Abbau zu einer Formel der folgenden allgemeinen Formel (IV):

(OHCHN)ₐ-X-(NCO)_{b} (IV)

worin a, b und X wie oben für Formel (I) definiert sind. Die Verbindung der Formel (IV) kann auch als Formamido-isocyanat bezeichnet werden. Die Thermolyse wird unter Bedingungen durchgeführt, die dem Fachmann für die Transformation von Carbonsäureaziden zu Isocyanaten herkömmlich bekannt sind (s. z.B. Organikum, loc.cit.). Die in der Thermolyse verwendete Temperatur beträgt vorzugsweise 60 bis 100°C. Insbesondere ist es bevorzugt, ein inertes Lösungsmittel zu verwenden, um eine weitere Reaktion der entstandenen Isocyanatogruppe(n) zu verhindern. Beispiele für das inerte Lösungsmittel sind wie oben für Methode A angegeben, bevorzugt Toluol, 1,4-Dioxan und Chlorbenzol.

In einem zweiten Schritt wird die Verbindung der Formel (IV) einer Dehydratisierung unterworfen, um die Formamidogruppe(n) in die Isocyanogruppe zu überführen. Die Dehydratisierung erfolgt vorzugsweise durch Phosgenierung wie in Methode A beschrieben, besonders bevorzugt bei einer Temperatur im Bereich von -80 bis 0°C. Die Dehydratisierung erfolgt bevorzugt in einem inerten Lösungsmittel, für das Beispiele in der Beschreibung der Methode A der vorliegenden Erfindung angegeben sind.

In einem dritten Verfahren (Methode C) wird direkt von einer Verbindung der allgemeinen Formel (IV) wie oben definiert ausgegangen, die einer Dehydratisierung wie in Methode B definiert unterworfen und dadurch zu einer Verbindung der Formel (I) umgesetzt wird. Die Reaktionsbedingungen in diesem dritten Verfahren sind daher wie für den zweiten Schritt der Methode B.

Je nach Reaktivität der funktionellen Gruppen der Verbindungen (II), (III) und (IV) und der Zielverbindung der Formel (I) kann es zweckmäßig sein, als Herstellungsverfahren für die Verbindung der Formel (I) Methode A der Methode B vorzuziehen oder umgekehrt. So ist es bekannt, daß die Phosgenierung von aromatischen Aminen zu aromatischen Isocyanaten allgemein höhere Temperaturen als die Phosgenierung einer Formamidogruppe zur Isocyanogruppe erfordert (i.d.R. >70°C für die aromatische Isocyanatogruppe gegenüber -40 bis +20°C für die Isocyanogruppe). Im Fall von Verbindungen der Formel (I), in denen die Isocyanatogruppe an einen Aromaten oder Heteroaromaten gebunden ist, kann es daher zweckmäßig sein, Methode B anstelle der Methode A zu verwenden und somit die Isocyanatogruppe durch Thermolyse (Curtius-Abbau) aus dem Carbonsäureazid zu erzeugen.

Die Methoden A und B werden exemplarisch in der folgenden Tabelle 1 anhand der Reaktionen (1) bis (4) dargestellt:

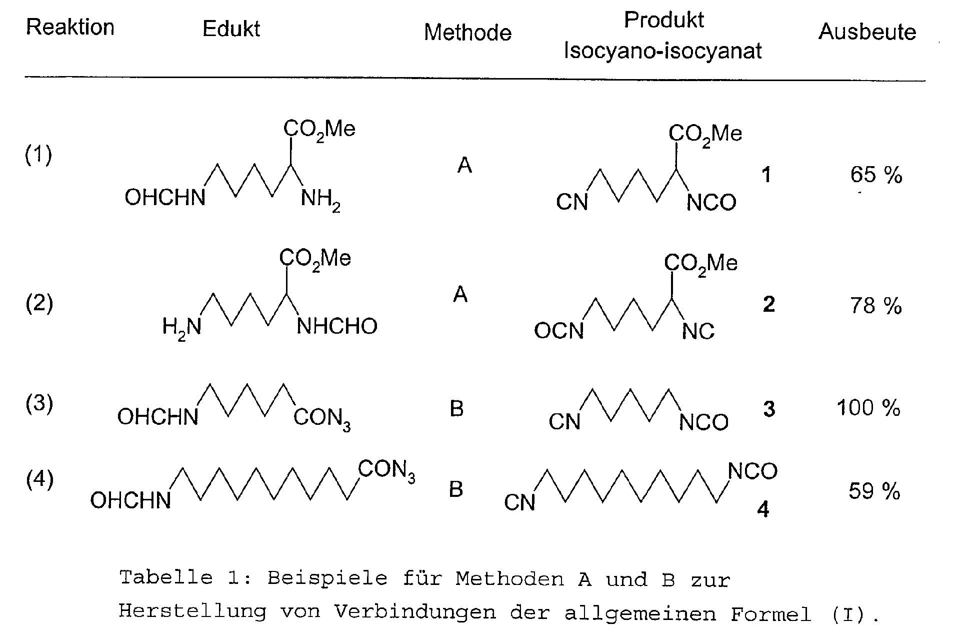

Die erfindungsgemäßen Verbindungen der allgemeinen Formel (I) haben sich allgemein als stabil erwiesen und können i.d.R. isoliert und bei Raumtemperatur gelagert werden. In speziellen Fällen können diese Verbindungen aber auch in Lösung gelagert oder, insbesondere bei einer geringeren Stabilität, in situ zu weiteren Produkten umgesetzt werden.

Die vorliegende Erfindung betrifft ebenfalls die Verwendung der Verbindungen der allgemeinen Formel (I) zur Herstellung weiterer (End-) Produkte. Verbindungen der Formel (I) können aufgrund der unterschiedlichen Selektivität der Isocyano- und Isocyanatogruppen selektiv mit Reagenzien umgesetzt werden. Solche Umsetzungen können in einer Eintopfreaktion erfolgen, wobei je nach ausgewähltem Reagenz nur Isocyano-, nur Isocyanato- oder beide Gruppen abreagieren. In einer besonderen Ausführungsform führt die selektive Reaktion der Isocyano- oder der Isocyanatogruppe (oder beider Gruppen) zu einer (oder zwei) intermediären Gruppe, die anschließend mit der jeweils anderen Gruppe (oder der jeweils anderen, intermediär gebildeten Gruppe) inter- oder intramolekular abreagiert.

Allgemein kann die Isocyanatogruppe in dem Fachmann bekannter Weise umgesetzt werden, z.B. mit einem Alkohol zum Urethan oder mit einem Amin zum Harnstoffderivat. Die Isocyanogruppe kann in dem Fachmann bekannter Weise umgesetzt werden, z.B. zu Heterocyclylresten wie Indolyl, Imidazolyl, Oxazolyl, Oxazolinyl, Oxazolidinyl, Pyrrolyl, Chinolinyl und Tetrazolyl (vgl. S. Marcaccini, T. Torroba, Org. Prep. Proceed. Int. 1993, 25, 141-208; D. Seebach et al., Chem. Ber. 1988, 121, 507-517).

Insbesondere können in einer Multikomponentenreaktion (MCR) die Isocyano-, die Isocyanatogruppe oder beide umgesetzt werden. Beispiele für solche Reaktionstypen sind z.B. die Passerine-Reaktion und die Ugi-Reaktion (s. z.B. A. Doemling, I. Ugi, Angew. Chemie Int. Ed. 2000, 39, 3168-3210).

Beispiele für die oben aufgeführten Reaktionen gemäß der erfindungsgemäßen Verwendung werden nachfolgend beschrieben.

Die folgende Tabelle 2 stellt eine allgemeine und nicht abschließende Übersicht zu selektiven Reaktionen von erfindungsgemäßen Verbindungen der Formel (I) dar:

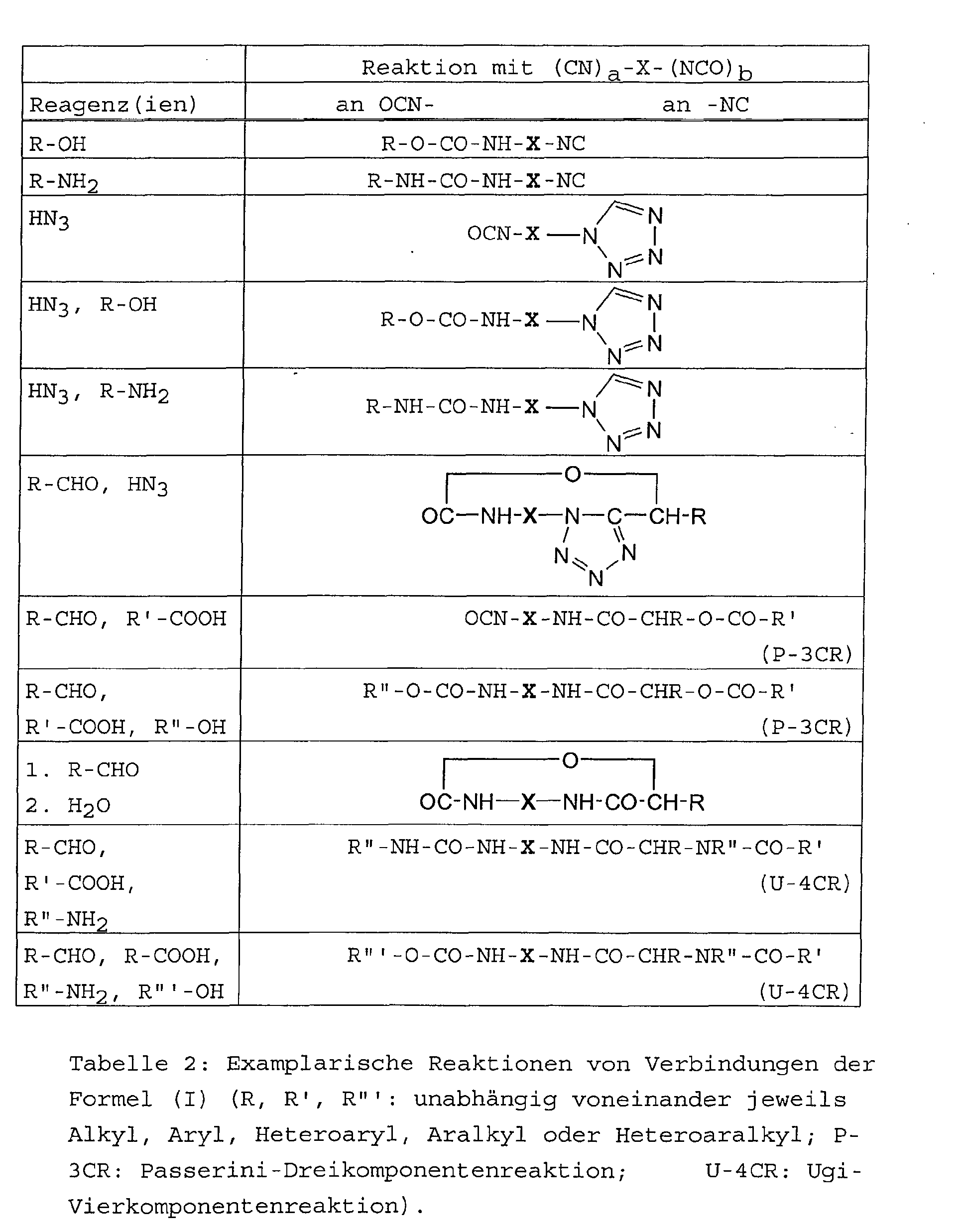

Gemäß der folgenden Reaktion (5) entsteht aus 1-Isocyanato-10-isocyano-decan mit Benzaldehyd, Essigsäure und Methanol in Dichlormethan aus der Isocyanogruppe in einer Passerini-Dreikomponentenreaktion (P-3CR) das Passerini-Produkt und aus der Isocyanatogruppe in einer Additionsreaktion das O-Methylurethan in 92 %iger Ausbeute. Reaktionsprodukte anderer Kombinationen der Isocyano- und Isocyanato-Gruppen mit den drei Reagenzien (Benzaldehyd, Essigsäure und Methanol) werden nicht beobachtet, d.h. die Reaktion verläuft hochselektiv. Damit können in einer einzigen Reaktionsstufe zwei Molekülteile in zwei unterschiedlichen Reaktionen gleichzeitig und selektiv umgesetzt werden. Der obige beispielhafte Vergleich mit der Aminosäure würde vier Reaktionsstufen erfordern, also drei zusätzliche Stufen.

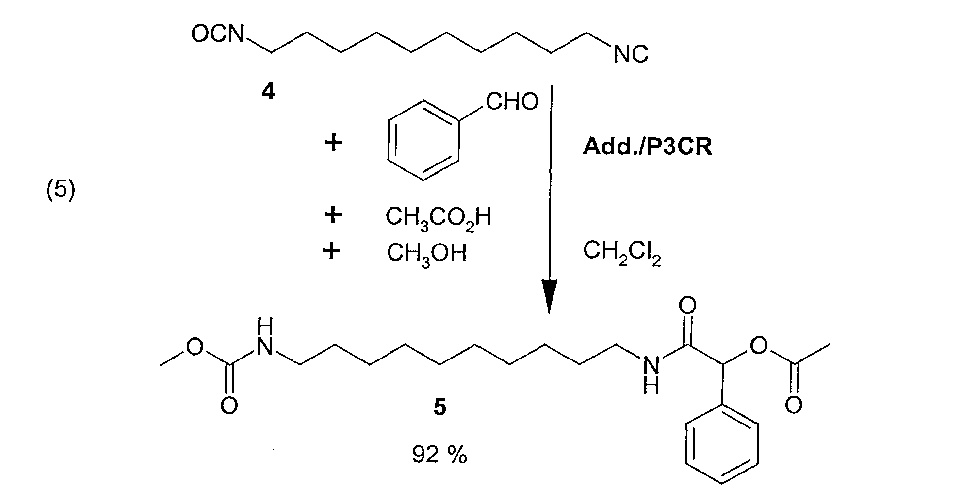

Damit wird gezeigt, daß in Synthesen, beispielsweise von wertvollen Wirkstoffen, mehrere Reaktionsstufen eingespart werden können, was in der Regel von hohem wirtschaftlichem Nutzen ist. Zudem werden empfindliche und wertvolle Zwischenstufen geschont, da sie für weitere Reaktionen nicht aktiviert werden müssen. Insgesamt stellen die erfindungsgemäßen Verbindungen der Formel (I) damit wertvolle Ausgangsstoffe für die chemische Synthese dar, insbesondere von Produkten, die Isocyanato- oder Isocyanogruppen oder daraus abgeleitete funktionelle Gruppen tragen. Als spezielle Gebiete kann hier die Verwendung von Verbindungen der Formel (I) in der Synthese von Polymerkomponenten oder -vorstufen, von pharmazeutischen Wirkstoffen und von Pestiziden, wie Insektiziden, Herbiziden und Fungiziden, genannt werden.

Eine weitere erfindungsgemäße Anwendung besteht in der Festphasentechnik. Dort stellt der einfache Zugang zu Isocyano-funktionalisierten Harzen nach wie vor ein Problem dar. Gemäß einer bevorzugten Ausführungsform der Erfindung liefert die Umsetzung von 1-Isocyanato-5-isocyanopentan mit hydroxymethyliertem Polystyrol in Dichlormethan z.B. nach Reaktion (6) in einer Stufe das Isocyano-funktionalisierte Polymer. Das Isocyanid kann dadurch über Urethan- oder Harnstoff-funktionen an einem Polymer immobilisiert werden und anschließend weiter umgesetzt werden, z.B. in MCRs wie oben erläutert.

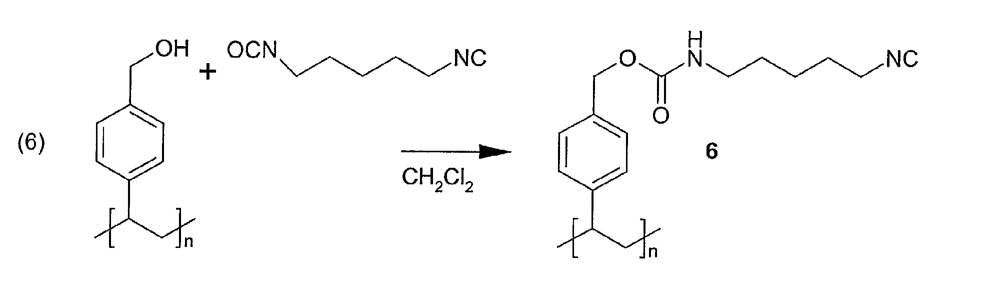

Somit können in einer bevorzugten Ausführungsform der Erfindung Isocyano-funktionalisierte Harze aus Hydroxyalkyloder Aminoalkyl-funktionalisierten Harzen und einer Verbindung der allgemeinen Formel (I) erhalten werden, insbesondere in einem einstufigen Prozeß.

### Reaktionsbeispiele

### Beispiel 1 (Methode A - Verbindung 1 aus Reaktion (1) in Tabelle 1)

21 g (0,11 mol) ε-Formyl-lysin-methylester und 74,5 g (0,74 mol) Triethylamin werden in 300 ml Dichlormethan gelöst und die Lösung auf -20°C gekühlt. Man leitet 25,8 g (0,26 mol) Phosgen bei dieser Temperatur innerhalb 45 min ein, läßt im (nicht weiter gekühlten) Kühlbad auf Raumtemperatur erwärmen und rührt insgesamt 12 h. Ein Vakuum wird angelegt, um überschüssiges Phosgen zu entfernen. Man gibt zum Reaktionsgemisch 150 ml trockenen Diethylether hinzu, filtriert das Reaktionsgemisch über Celite und engt das Filtrat im Vakuum ein. Der Rückstand wird im Hochvakuum destilliert. Man erhält 14 g (65 %) einer farblosen Flüssigkeit, die IR- und NMR-spektroskopisch als Verbindung 1 (s. Tabelle 1, Reaktion (1)) nachgewiesen wird.
ν (N=C=O) = 2260 cm⁻¹, ν (N=C) = 2140 cm⁻¹, ν (C=O) = 1730 cm⁻¹

### Beispiel 2 (Umsetzung von Verbindung 4 zu Verbindung 5 in Reaktion (5))

Man legt 0,5 ml (9,3 mmol) Methanol in 5 ml Dichlormethan vor und versetzt bei Raumtemperatur nacheinander mit 260 mg (2,5 mmol) Benzaldehyd, 150 mg (2,5 mmol) Essigsäure und 500 mg (2,5 mmol) 1-Isocyanato-10-isocyanodecan (Verbindung 4 in Tabelle 1). Nach 50 h wird die Reaktionslösung im Vakuum eingeengt, wobei der Rückstand vollständig kristallisiert. Man erhält 920 mg (92 % Ausbeute) eines Produkts, das NMR-spektroskopisch als das gewünschte Urethan/Passerini-Produkt (Verbindung 5) nachgewiesen wird.
¹H-NMR (60 MHz): δ = 1,17 (s, 8), 2,12 (s, 3), 3,16 (t, 4), 3,61 (s, 3), 4,85 (br, 1) 6,07 (s, 1), 6,34 (br, 1), 7,36 (s, 5)

### Beispiel 3 (Herstellung des Polymers 6 in Reaktion (6))

600 mg Poly(hydroxymethylstyrol) (Beladung 1,24 mmol Hydroxymethyl pro Gramm) werden in 4 ml Dichlormethan bei Raumtemperatur 15 h lang quellen gelassen. Dann gibt man 100 mg (0,72 mmol) 1-Isocyanato-5-isocyanopentan hinzu und rührt 5 Tage bei Raumtemperatur. Eine analytische Probe wird entnommen, abfiltriert, mit Dichlormethan gewaschen und IRspektroskopisch als Polymer 6 nachgewiesen.
ν (N=C) = 2130 cm⁻¹, ν (C=O) = 1710 cm⁻¹

## Patentansprüche

1. Verbindung der folgenden allgemeinen Formel (I)
(CN)ₐ-X-(NCO)_{b} (I)
worin a und b unabhängig voneinander eine ganze Zahl ≥ 1 sind und X ausgewählt ist aus:
- geradkettigem oder verzweigtkettigem Alkyl, gegebenenfalls mit einer oder mehreren Gruppen in der Alkylkette, unabhängig ausgewählt aus Cycloalkyl, Aryl und Heterocyclyl,
- Cycloalkyl,
- Aryl und
- Heterocyclyl,
jeweils gegebenenfalls substituiert mit einer oder mehreren funktionellen Gruppen, die von Isocyano und Isocyanato verschieden sind.

2. Verbindung gemäß Anspruch 1, worin a und b unabhängig voneinander eine ganze Zahl im Bereich von 1 bis 4 sind, vorzugsweis jeweils 1.

3. Verbindung gemäß Anspruch 1 oder 2, worin X geradkettiges oder verzweigtkettiges C₃₋₂₅-Alkyl ist, bevorzugt C₅₋₁₂-Alkyl, besonders bevorzugt C₅-Alkyl.

4. Verbindung gemäß einem der Ansprüche 1 bis 3, worin Cycloalkyl als X oder Teil von X C₃₋₁₀-Cycloalkyl ist, bevorzugt C₅₋₈-Cycloalkyl, besonders bevorzugt C₆-Cycloalkyl.

5. Verbindung gemäß einem der Ansprüche 1 bis 4, worin Aryl als X oder Teil von X eine mono-, bi- oder polycyclische Arylgruppe ist, vorzugsweise eine 6-gliedrige Arylgruppe.

6. Verbindung gemäß einem der Ansprüche 1 bis 5, worin Heterocyclyl als X oder Teil von X eine mono-, bi- oder polycyclische, aromatische oder nicht-aromatische Heterocyclylgruppe ist, worin jeder Ring vorzugsweise 4-, 5-oder 6-gliedrig ist und ein oder mehrere Heteroatome aus N, O und S enthält.

7. Verbindung gemäß einem der Ansprüche 1 bis 6, worin die eine oder mehreren funktionellen Gruppen unabhängig ausgewählt sind aus einer Estergruppe, einer Säureamidgruppe, einer Ketogruppe, einer Nitrogruppe, einem Halogenatom und einer Cyanogruppe.

8. Verbindung gemäß einem der Ansprüche 1 bis 7, worin die eine oder mehreren funktionellen Gruppen gegenüber Isocyano oder Isocyanato oder beiden nicht reaktiv sind.

9. Verfahren zur Herstellung einer Verbindung der allgemeinen Formel (I) wie in einem der Ansprüche 1 bis 8 definiert, umfassend die Phosgenierung einer Verbindung der folgenden allgemeinen Formel (II):
(OHCHN)ₐ-X-(NH₂)_{b} (II)
worin a, b und X wie hier oben definiert sind.

10. Verfahren gemäß Anspruch 9, worin die Reaktion bei einer Temperatur im Bereich von -40 bis +40°C durchgeführt wird.

11. Verfahren zur Herstellung einer Verbindung der allgemeinen Formel (I) wie in einem der Ansprüche 1 bis 8 definiert, umfassend die Thermolyse einer Verbindung der folgenden allgemeinen Formel (III):
(OHCHN)ₐ-X-(CON₃)_{b} (III)
unter Erhalt einer Verbindung der folgenden allgemeinen Formel (IV):
(OHCHN)ₐ-X-(NCO)_{b} (IV)
und Dehydratisierung der Verbindung der allgemeinen Formel (IV) unter Erhalt der Verbindung der allgemeinen Formel (I), worin a, b und X wie hier oben definiert sind.

12. Verfahren gemäß Anspruch 11, worin die Thermolyse bei einer Temperatur im Bereich von 60 bis 100°C durchgeführt wird.

13. Verfahren zur Herstellung einer Verbindung der allgemeinen Formel (I) wie in einem der Ansprüche 1 bis 8 definiert, umfassend die Dehydratisierung einer Verbindung der folgenden allgemeinen Formel (IV):
(OHCHN)ₐ-X-(NCO)_{b} (IV)
unter Erhalt der Verbindung der allgemeinen Formel (I), worin a, b und X wie hier oben definiert sind.

14. Verfahren gemäß einem der Ansprüche 11 bis 13, worin die Dehydratisierung durch Phosgenierung durchgeführt wird.

15. Verfahren gemäß einem der Ansprüche 11 bis 14, worin die Dehydratisierung bei einer Temperatur im Bereich von -80 bis 0°C durchgeführt wird.

16. Verfahren gemäß einem der Ansprüche 11 bis 15, worin die Dehydratisierung in einem inerten Lösungsmittel durchgeführt wird.
